# EUROPEAN PATENT SPECIFICATION

(11) **EP 1 099 434 B1**
(45) Date of publication and mention of the grant of the patent: **07.04.2004**
(21) Application number: 00115484.8
(22) Date of filing: 18.07.2000
(51) Int. Cl.: A61F 13/15, D04H 11/00, B26F 1/26

(54) **Nonwoven material comprising an adhesive and apertures**
Vliesstoff mit Klebstoff und Öffnungen
Matériau non-tissé comprenant un adhésif et des ouvertures

(30) Priority: 09.11.1999 US 436603
(43) Date of publication of application: 16.05.2001
(73) Proprietor: McNEIL-PPC, INC., Skillman, New Jersey 08558 (US)
(72) Inventor: Lasko, Vincent P., New Egypt, NJ 08533 (US)
(74) Representative: Groening, Hans Wilhelm, Dipl.-Ing.

(56) References cited:
- EP-A- 0 737 462
- EP-A- 0 861 646
- US-A- 3 967 623
- US-A- 4 324 246
- US-A- 5 620 742
- US-A- 5 643 237
- DATABASE WPI Section Ch, Week 197721 Derwent Publications Ltd., London, GB; Class A32, AN 1977-37178Y XP002162814 & JP 52 047080 A (OTSUKA M), 14 April 1977 (1977-04-14)

## Description

The present invention relates to a nonwoven material having apertures and an adhesive composition disposed in a pattern on surface thereof, a process for making the same, and absorbent articles comprising the same.

### Background of the Invention

Many types of nonwoven materials, that is, fibrous nonwoven webs or plastic films, find useful application in the field of absorbent articles. Such materials may or may not be apertured, and are often used as covers or backsheets for sanitary napkins, pantiliners, diapers, incontinence devices and wound dressings. In order to enhance the functional and aesthetic properties of these materials, they are designed with a variety of features.

For example, U.S. Patent No. 5,906,786 relates to the production of nonwoven fabrics with fibrous raised portions and fibrous background portions. The basis weights and densities of the raised and background portions can be adjusted to achieve differing effects. U.S. Patent No. 5,824,352 describes an apparatus for producing an apertured plastic film having a tricot-like texture due to a series of peaks and valleys in the film. U.S. Patent No. 5,736,219 relates to a nonwoven fabric having improved absorbent characteristics. The fabric has three different fiber arrays that are interconnected to produce a unique fiber distribution in the fabric.

The European Patent application EP 0 737 462 A 1 discloses a laminated material intended to cover the outside of an absorbent product, characterised in that at least one portion of the surface of that laminated material bears a layer of fibres applied by flocking. The laminated material is preferable produced on a perforated plastic film.

Applicant has now discovered that a nonwoven material comprising a combination of apertures and, in a preferred embodiment patterned land areas, may be formed by applying an adhesive composition to the nonwoven material and aperturing it. Such a nonwoven material is particularly useful as a component of an absorbent article. Advantageously, the nonwoven material may be made with a variety of functional or aesthetic properties by adjusting the ingredients of the adhesive composition and the pattern in which it is applied to the nonwoven material.

### Summary of the Invention

The invention provides a process for preparing a nonwoven material having a surface and a second surface opposite the first surface, which comprises in sequence:
a) applying an adhesive composition to the first surface of the nonwoven material in a pattern; b) curing the adhesive composition; and c) aperturing the adhesive composition containing nonwoven material characterised in that, said aperturing forms a plurality of apertures originating in said second surface of the nonwoven material, such that side walls of said apertures protrude away from the second surface, projecting outward from the first surface.

The invention further provides a nonwoven material comprising: 1) a first surface and a second surface opposite the first surface, 2) an adhesive composition disposed in a pattern on the first surface of the nonwoven material, and 3) a plurality of apertures in said nonwoven material, characterised in that, said plurality of apertures originate in said second surface of the nonwoven material, such that side walls of said apertures protrude away from the second surface, projecting outward from the first surface.

The invention also provides an absorbent article comprising a nonwovenmaterial and an absorbent core, said nonwoven material comprising: 1) a first surface and a second surface opposite the first surface, 2) an adhesive composition disposed in a pattern on the first surface of the nonwoven material, and 3) a plurality of apertures in said nonwovenmaterial, characterised in that, said plurality of apertures originate in said second surface of the nonwovenmaterial, such that side walls of said apertures protrude away from the second surface, projecting outward from the first surface.

### Brief Description of the Drawings

Figure 1 is a cross-sectional view of a nonwoven material according to the invention.
Figure 2 is a top view of the nonwoven material of Figure 1.
Figure 3 is a bottom view of the nonwoven material of Figure 1.
Figure 4 depicts a pantiliner comprising a cover made from a nonwoven material according to the invention.

### Detailed Description of the Invention

Production of the nonwoven material starts with any textile-like material that is not a woven fabric. In particular, fibrous nonwoven webs that are formed by carding or hydroentangling processes are useful starting materials. Plastic films, such as those comprising polyethylene, polypropylene, or cellophane may also be used. A wide range of examples of fibrous nonwoven webs and plastic films are known and used in the art of absorbent articles, and any of these may be employed. The identity of the starting material is not critical to the invention.

The nonwoven material is made by first applying an adhesive composition to one surface of the nonwoven material in a pattern. The adhesive composition comprises an adhesive such as a solution or emulsion based acrylic polymer synthesized from one or more acrylic or methacrylic ester monomers such as 2-ethylhexyl acrylate, n-butyl acrylate, iso-octyl acrylate, 1-decyl acrylate, acrylic acid, methacrylic acid, N-methylacrylamide, ethyl acrylate, methyl acrylates, methyl methacrylate, ethyl methacrylate, and butyl methacrylate. Such adhesives may also include vinyl based comonomers such as vinyl acetate, vinyl chloride, styrene, maleic anhydride, and crotonic acid. The adhesives may also be crosslinked via vinyl monomers that contain pendent groups such as amide, carboxyl, hydroxyl, or epoxy functionality.

Commercially available examples of useful adhesives include FLEXBOND 974, 977, 983, and 986 adhesives available from Air Products, CARBOTAC Adhesives (pressure sensitive adhesives) available from BF Goodrich, and CARBOBOND Adhesives (non-pressure sensitive adhesives) also available from BF Goodrich. Preferably, the adhesive is selected from the group consisting of FLEXBOND 974, 977, 983, and 986 adhesives.

The adhesive composition also preferably comprises an additive for the purpose of enhancing the functional (for example, absorbent) properties of the nonwoven material or effecting the aesthetic nature of the nonwoven material. Such additive may be added to the adhesive composition in solid or liquid form. Preferably, the additive is added to the adhesive composition in solid form, such as in the form or fibers or particles.

In a particularly preferred embodiment of the invention, the adhesive composition comprises an additive in the form of flocked fibers. Methods of flocking fibers are known in the art of fabric manufacture. See for example, U.S. Patent Nos. 2,527,501; 2,691,611; 3,436,442; and 3,672,929. In the present invention, the flocked fibers are added to the adhesive composition after it has been applied to the surface of the nonwoven material. The nonwoven material containing the adhesive composition is passed through a fiber metering station in which an electrostatic field is maintained around the nonwoven material, using for example electrodes situated above and below the nonwoven material. The fibers are applied to the adhesive composition on the nonwoven material in the presence of the electrostatic field, which orients the fibers perpendicular to the nonwoven material as they contact the adhesive composition. The nonwoven material is then heated, polymerizing the adhesive and anchoring the fibers in the adhesive composition. Unattached fibers may be vacuumed away.

In general, any additive in fiber form may be flocked onto the adhesive composition as described above. Typically, the length of the flocked fibers should be less than about 1 mm, preferably less than about 0.8 mm. The denier of the flocked fibers should be in the range of about 1.2d to about 15d, preferably about 1.8d to about 6d.

Generally, the additive may be selected from hydrophilic materials, including wettable materials and absorbents, hydrophobic materials such as repellants, superabsorbent polymers, pigments, and combinations thereof.

Examples of wettable materials include bicomponent fibers, polypropylene fibers, and polyester fibers that have been treated for example with surfactants. Preferred wettable materials are polyester fibers, such as DuPont-Akra Polyester Type 11A Bright commercially available from DuPont Company treated with a surfactant such as Tween 20 commercially available from ICI Americas Inc.

Absorbents both have an affinity for and absorb fluids. Absorbents are typically also used in fiber form, and may comprise rayon, acrylics, nylon, polyvinyl alcohol, and natural or regenerated cellulosics. A preferred type of absorbent is rayon fibers.

Hydrophobic materials include certain olefins and polyesters, preferably used in fiber form and having a denier of at least 3, more preferably at least 6. A preferred hydrophobic fiber is 15 denier polyester commercially available from DuPont Company. In another particularly preferred embodiment of the invention, the adhesive composition comprises superabsorbent polymer, which may be in either particle or fiber form. Preferably, the superabsorbent polymer is in fiber form, more preferably in the form of flocked fibers. Superabsorbent polymers are hydrophilic materials that are swellable and capable of absorbing greater than about 5 grams per gram (of fiber weight) of 1% saline solution. Examples of superabsorbent polymers are known in the art and include polyacrylates, grafted cellulose, and maleic acid. Preferred types of superabsorbent polymer fibers include OASIS Type 101, commercially available from Technical Absorbents Limited and CAMELOT, commercially available from Camelot, Alberta, Canada. Examples of superabsorbent polymers in particle form include ASAP 2000, ASAP 2300, and ASAP 2100A commercially available from Chemdal Corp., Aquakeep J-550 and Aquakeep SA60N type 2 commercially available from Sumitomo, and SXM-4750 commercially available from Stockhausen.

Pigments useful in the adhesive composition include titanium dioxide, calcium carbonate, and other whiteners, for example. Pigments of other colors may also be used.

The adhesive composition is applied to one surface of the nonwoven material in a pattern. Any pattern may be used. One preferred pattern involves applying the adhesive composition around the perimeter of the surface of the nonwoven material to form a barrier of sorts along the edge of the nonwoven material. For example, an adhesive composition comprising adhesive mixed with hydrophobic material (in fiber or particle form) applied around the perimeter of the surface of the nonwoven material decreases fluid flow across the adhesive composition, thereby preventing fluid flow off the edge of the nonwoven material.

Other patterns for application of the adhesive composition may be partially or fully decorative, such a pattern of individual flowers, stars, etc. dispersed over all or part of the surface of the nonwoven material.

The adhesive composition is then cured, typically by the application of heat. After curing of the adhesive composition, the nonwoven material is apertured. The invention is not limited to any particular aperturing process. Aperturing of nonwoven materials is well known in the art, and any technique may be used according to the invention. Generally speaking, a starting material, in this case the nonwoven material comprising the adhesive composition, is placed over the surface of a support member comprising a plurality of holes, and optionally other topographical features. High pressure fluid such as water or air is then directed against the nonwoven material, which causes it to conform to the topography of the support member. Alternatively, a vacuum may be drawn from underneath the support member, also causing the nonwoven material to conform to the surface of the support member. U.S. Patent Nos. 4,342,314, 5,366,782, 5,916,462 and 5,824,352 disclose the aperturing process and a variety of support members in greater detail.

Aperturing may be done so that the apertures originate in the surface of the nonwoven material on which the adhesive composition has been applied. Alternatively, the apertures may originate on the surface of the nonwoven material that is opposite to the surface on which the adhesive composition has been applied. Preferably, the apertures originate on the surface of the nonwoven material that is opposite to the surface on which the adhesive composition has been applied. Apertures "originate" in a surface of a planar material when their sidewalls protrude away from such surface, projecting outward from the opposite surface.

Aperturing of the nonwoven material may be performed as desired such that holes are or are not also formed in the areas of the nonwoven material in which the adhesive composition has been applied. Preferably, aperturing is performed such that holes are not formed in the areas of the nonwoven material in which the adhesive composition has been applied. In this manner, the pattern in which the adhesive composition has been applied is substantially maintained. Indeed, the pattern of the adhesive composition may even be enhanced by aperturing without forming holes in the areas in which the adhesive composition has been applied. Depending on the temperature of the nonwoven material and the pressure exerted against the nonwoven material during the aperturing step, the areas of the nonwoven material containing the adhesive composition may actually become raised relative to the areas not containing adhesive composition.
Advantageously, an overall effect similar to embossing is achieved and functional and/or decorative patterns may be imparted to an apertured film using relatively straightforward techniques.

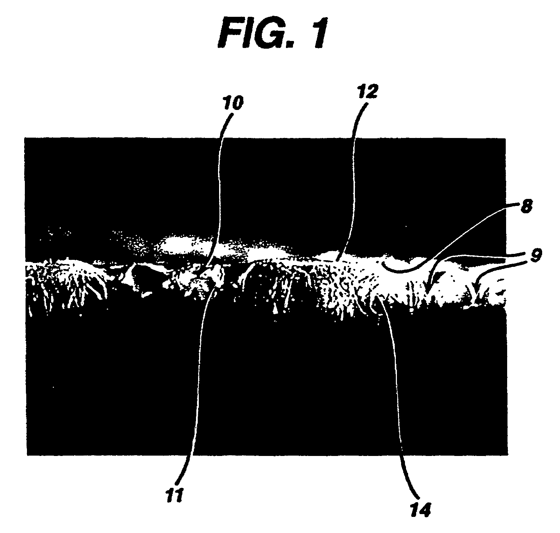

Figure 1 shows a cross-sectional view of a nonwoven material according to the invention in which aperturing has been performed without producing holes in the areas in which the adhesive composition has been applied. The nonwoven material of Figure 1 comprises a plastic film 10 having a first surface 11 and a second surface 12 with an adhesive composition 8 disposed in a pattern on the first surface 11 thereof. The adhesive composition contains adhesive with flocked fibers 14 of an additive such as a superabsorbent polymer projecting therefrom. The plastic film 10 also contains a plurality of apertures 9. The apertures 9 originate in the second surface 12 of the plastic film 10.

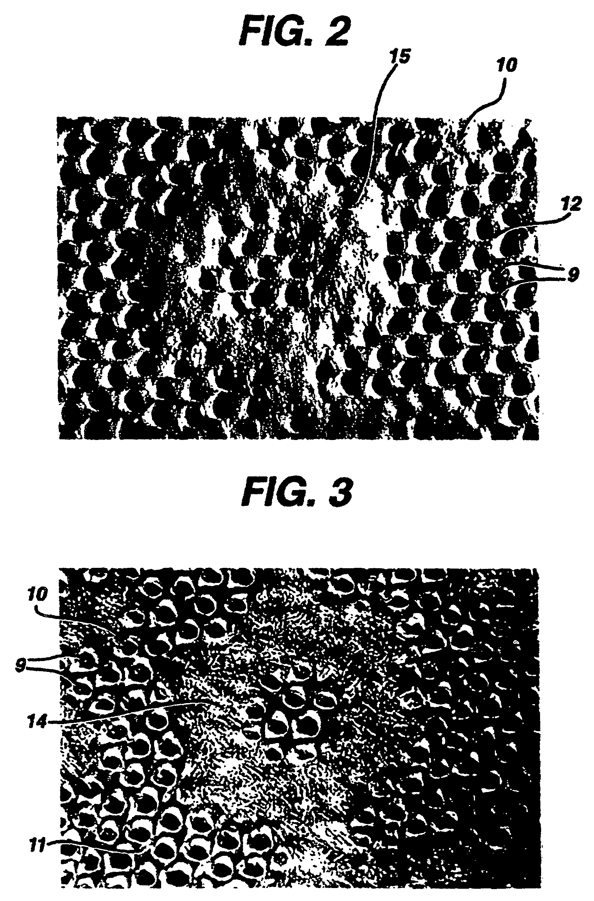

Figure 2 is a top view of the nonwoven material of Figure 1. The second surface 12 of the plastic film 10 comprises a series of small land areas surrounding the apertures 9. In addition, a large land area 15 is present where the adhesive composition has been applied to the first surface 11 (not shown).

Figure 3 shows a bottom view of the nonwoven material of Figure 1. The sidewalls of the apertures 9 project outward from the first surface 11 of the plastic film 10. The flocked fibers 14 also project outward from the adhesive composition and the first surface 11.

The nonwoven material may be used in an absorbent article, for example as a cover or a backsheet. Such absorbent article may be a sanitary napkin, a pantiliner, a diaper, incontinence pad, interlabial article, or other similar product for absorbing exudates from the body, such as menses, urine, or feces. Preferably, the absorbent article is a sanitary napkin or a pantiliner. Such sanitary napkin or pantiliner may have an approximately rectangular, oval, dogbone, or peanut shape. Depending on the nature of the absorbent article, its size may vary. For example, sanitary napkins typically have a caliper of about 1.4 to about 5 mm, a length of about 7,62 cm to about 40,64 cm (3 to about 16 inches), and a width of about 2,54 cm to about 12,7 cm (1 to about 5 inches). Pantiliners typically have a caliper of less than about 0,508 cm (0.2 inches), a length of less than about 20,23cm (8 inches), and a width of less than about 7,62 cm (3 inches).

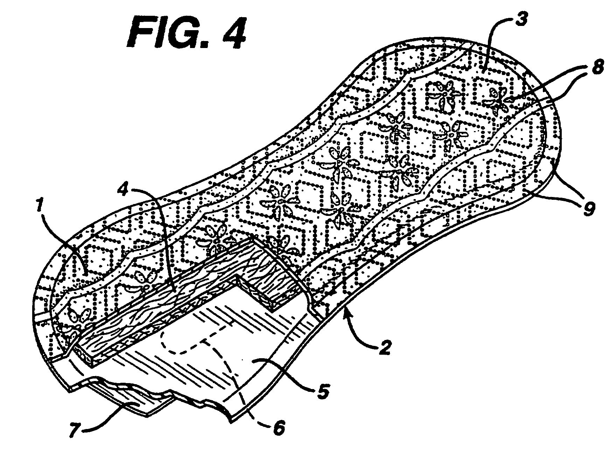

Figure 4 depicts a pantiliner in which the cover is a nonwoven material according to the invention, and is used for purposes of illustration in the following description. The pantiliner shown in Figure 4 comprises in sequence from its body-facing surface 1 to its garment-facing surface 2 liquid permeable cover 3, an absorbent core 4, and a liquid impermeable backsheet 5. The cover 3 of the absorbent article is made from a plastic film that comprises an adhesive composition 8 and a plurality of apertures 9. In this case, the adhesive composition is disposed in a pattern of rails and flowers on the side of the cover 3 facing the absorbent core 4. The apertures 9 originate in the body-facing surface 1 of the cover and project downward towards the absorbent core 4.

In general, the thickness of the cover 3 may vary from approximately 0,0254 mm to 1,5748 mm (0.001 to 0.062 inch), depending on the material chosen. The cover may be the same length, or optionally longer than the absorbent core so as to form transverse ends. Such transverse ends may be sealed with other layers to fully enclose the absorbent core.

The absorbent core 4 may be comprised of a loosely associated absorbent hydrophilic material such as cellulose fibers, including wood pulp, regenerated cellulose fibers or cotton fibers, or other absorbent materials generally known in the art, including acrylic fibers, polyvinyl alcohol fibers, peat moss and superabsorbent polymers.

The absorbent article further comprises a liquid impermeable backsheet 5, the exterior of which forms the garment-facing surface 2 of the article. The backsheet may comprise any thin, flexible, body fluid impermeable material such as a polymeric film, for example, polyethylene, polypropylene, or cellophane. Alternatively, the backsheet may be a normally fluid permeable material that has been treated to be impermeable, such as impregnated fluid repellent paper or non-woven fabric material, or a flexible foam, such as polyurethane or cross-linked polyethylene. The thickness of the backsheet when formed from a polymeric film typically is about 0,0254 mm to 0,0508 mm (0.001 to 0.002 inch). A variety of materials are known in the art for use as backsheet, and any of these may be used.

Generally, the backsheet 5 is a single sheet of material having a width sufficient to form the garment-facing surface 2 of the absorbent article. The backsheet may extend around the sides of the absorbent core in a C-shaped configuration with the portions of the backsheet adjacent its longitudinal edges extending upwardly from the garment-facing surface toward the body-facing surface of the article. Preferably the backsheet is breathable, i.e., a film that is a barrier to liquids but permits gases to transpire. Materials for this purpose include polyurethane films and microporous films in which microporosity is created by ionizing radiation or by leaching out of soluble inclusions using aqueous or nonaqueous solvents. Single or multiple layers of permeable films, fabrics, and combinations thereof that provide a tortuous path, and/or whose surface characteristics provide a liquid surface repellent to the penetration of liquids may also be used to provide a breathable backsheet.

The absorbent article may be applied to the crotch of underpants by placing the garment-facing surface 2 of the absorbent article against the inside surface of the crotch of the underpants. Strips of pressure sensitive adhesive 6 may be applied to the garment-facing surface 2 of the absorbent article to help maintain it in place. As used herein, the term "pressure-sensitive adhesive" refers to any releasable adhesive or releasable tenacious means. Suitable pressure sensitive adhesives include for example water-based adhesives such as acrylate adhesives. Alternatively, the adhesive may comprise rapid setting thermoplastic "hot melt" rubber adhesives or two-sided adhesive tape.

A paper release strip 7 that has been coated on one side may be applied to protect the strips of adhesive 6 prior to use. The coating, for example silicone, reduces adherence of the coated side of the release strip to the adhesive. The release strip can be formed from any suitable sheet-like material which, when coated, adheres with sufficient tenacity to the adhesive to remain in place prior to use but can be readily removed when the absorbent article is to be used.

The absorbent article may comprise other known materials, layers, and additives, such as transfer layers, foam layers, net-like layers, odor control agents, perfumes, medicaments, moisturizers, and the like, many examples of which are known in the art.

### Example

A nonwoven material is made according to the invention as follows. A sample of polyester fabric or polyethylene film is used as the starting material. An adhesive composition containing titanium dioxide is applied to one surface of the starting material in a pattern of flowers and rails, similar to the pattern shown in Figure 4. Flocked fibers of superabsorbent polymer and polyester are metered onto the adhesive composition and then oriented in the vertical position via an electrostatic field. The fibers adhere where the adhesive composition has been applied. The material is then passed through an oven to polymerize the adhesive composition, anchoring the flocked fibers thereto. Excess fibers are vacuumed away.

Next, the material is apertured using hot air aperturing. The apertures originate in the surface of the material opposite to the one on which the adhesive composition has been applied. The temperature of the nonwoven material and the pressure of the hot air against the surface of the nonwoven material are adjusted so that apertures are not formed in the flowers and rails of the adhesive composition. The overall look and feel of the finished nonwoven material resembles a combination of apertures and embossed land areas.

## Claims

1. A process for preparing a nonwoven material having a first surface (11) and a second surface (12) opposite the first surface (11), which comprises in sequence:
a) applying an adhesive composition (8) to the first surface (11) of the nonwoven material in pattern;
b) curing the adhesive composition (8); and
c) aperturing the adhesive composition-containing nonwoven material;
**characterised in that**, said step of aperturing comprises a step of forming a plurality of apertures (9) originating in said second surface (12) of the nonwoven material, wherein side walls of said apertures (9) are shaped to protrude away from the second surface (12), projecting outward from the first surface.

2. The process of claim 1, wherein said aperturing is performed such that said apertures (9) are formed in the areas of the nonwoven material in which the adhesive composition (8) has not been applied.

3. The process of claim 1 or 2, wherein said aperturing is performed such that none of said apertures but embossed land areas (15) are formed in the areas of the nonwoven material in which the adhesive composition (8) has been applied, the embossed land areas becoming raised relative to areas in which the adhesive composition (8) has not been applied.

4. The process of claim 1, wherein the nonwoven material is a fibrous nonwoven web or plastic film (10).

5. The process of claim 1, wherein the adhesive composition (8) comprises an additive selected from the group consisting of hydrophilic materials, hydrophobic materials, superabsorbent polymers, pigments, and combinations thereof.

6. A nonwoven material comprising:
1) a first surface (11) and a second surface (12) opposite the first surface (11),
2) an adhesive composition (8) disposed in a pattern on the first surface (11) of the nonwoven material, and
3) a plurality of apertures (9) in said nonwoven material,
**characterised in that**, said plurality of apertures (9) originate in said second surface (12) of the nonwoven material, such that side walls of said apertures (9) protrude away from the second surface (12), projecting outward from the first surface.

7. The nonwoven material of claim 7, wherein the nonwoven material comprises the plurality of apertures (9) in areas, in which the adhesive composition (8) has not been applied.

8. The nonwoven material of claim 8, wherein the nonwoven material comprises no holes but embossed land areas (15) in areas, in which the adhesive composition (8) has been applied, the embossed land areas (15) being raised relative to the areas of the nonwoven material, in which the adhesive composition (8) has not been applied.

9. The nonwoven material according to any one of the claims 7 to 9, wherein the non-woven material is a fibrous nonwoven web or plastic film (10).

10. The nonwoven material according to any one of the claims 7 to 9, wherein the adhesive composition (8) comprises an additive selected from the group consisting of hydrophilic materials, hydrophobic materials, superabsorbent polymers, pigments and combinations thereof.

11. An absorbent article comprising an nonwoven material according to any one of the claims 7 to 11 and an absorbent core (4).

12. The absorbent article of claim 12, wherein said nonwoven material is the cover (3) of the absorbent article.

13. The absorbent article of claim 12, wherein said nonwoven material is the backsheet (5) of the absorbent article.

## Patentansprüche

1. Verfahren zum Herstellen eines nicht gewobenen Materials mit einer ersten Oberfläche (11) und einer zweiten Oberfläche (12) gegenüberliegend der ersten Oberfläche (11), das in folgender Reihenfolge die Schritte aufweist:
a) Auftragen einer Klebstoffverbindung (8) auf die erste Oberfläche (11) des nicht gewobenen Materials in einem Muster;
b) Aushärten der Klebstoffverbindung (8); und
c) Öffnen der Klebstoffverbindung, die nicht gewobenes Material enthält; **dadurch gekennzeichnet, daß**
der Schritt des Öffnens einen Schritt eines Bildens einer Mehrzahl von Öffnungen (9) umfaßt, die ihren Ursprung in der zweiten Oberfläche (12) des nicht gewobenen Materials haben, wobei Seitenwände der Öffnungen (9), die von der ersten Oberfläche nach außen herausragen, so geformt sind, daß sie von der zweiten Oberfläche (12) weg hervorragen.

2. Verfahren nach Anspruch 1, wobei das Öffnen so durchgeführt wird, daß die Öffnungen (9) in den Bereichen des nicht gewobenen Materials gebildet werden, in denen die Klebstoffverbindung (8) nicht aufgetragen wurde.

3. Verfahren nach Anspruch 1 oder 2, wobei das Öffnen so durchgeführt wird, daß keine der Öffnungen, sondern geprägte Landflächen (15) in den Bereichen des nicht gewobenen Materials gebildet werden, in denen die Klebstoffverbindung (8) aufgetragen wurde, wobei die geprägten Landflächen im Verhältnis zu Bereichen, in welchen die Klebstoffverbindung (8) nicht aufgetragen wurde, angehoben werden.

4. Verfahren nach Anspruch 1, wobei das nicht gewobene Material ein faserförmiges nicht gewobenes Gewebe oder ein Kunststoffilm (10) ist.

5. Verfahren nach Anspruch 1, wobei die Klebstoffverbindung (8) einen Zusatz umfaßt, der ausgewählt ist aus der Gruppe bestehend aus hydrophilen Materialien, hydrophoben Materialien, superabsorbierenden Polymeren, Pigmenten und Kombinationen daraus.

6. Nicht gewobenes Material, welches aufweist:
1) eine erste Oberfläche (11) und eine zweite Oberfläche (12) gegenüberliegend der ersten Oberfläche (11),
2) eine Klebstoffverbindung (8), die in einem Muster auf der ersten Oberfläche (11) des nicht gewobenen Materials angeordnet ist, und
3) eine Mehrzahl von Öffnungen (9) in dem nicht gewobenen Material,
**dadurch gekennzeichnet, daß** die Mehrzahl von Öffnungen (9) ihren Ursprung in der zweiten Oberfläche (12) des nicht gewobenen Materials hat, so daß die Seitenwände der Öffnungen (9), die von der ersten Oberfläche nach außen herausragen, von der zweiten Oberfläche (12) weg hervorragen.

7. Nicht gewobenes Material nach Anspruch 6, wobei das nicht gewobene Material die Mehrzahl von Öffnungen (9) in Bereichen aufweist, in welchen die Klebstoffverbindung (8) nicht aufgetragen wurde.

8. Nicht gewobenes Material nach Anspruch 7, wobei das nicht gewobene Material keine Löcher sondern geprägte Landflächen (15) in Bereichen aufweist, in denen die Klebstoffverbindung (8) aufgetragen wurde, wobei die geprägten Landflächen (15) im Verhältnis zu den Bereichen des nicht gewobenen Materials, in welchen die Klebstoffverbindung (8) nicht aufgetragen wurde, erhöht sind.

9. Nicht gewobenes Material nach einem der Ansprüche 6 bis 8, wobei das nicht gewobene Material ein faserförmiges nicht gewobenes Gewebe oder ein Kunststoffilm (10) ist.

10. Nicht gewobenes Material nach einem der Ansprüche 6 bis 9, wobei die Klebstoffverbindung (8) einen Zusatz umfaßt, der aus der Gruppe ausgewählt ist, bestehend aus hydrophilen Materialien, hydrophoben Materialien, superabsorbierenden Polymeren, Pigmenten und Kombinationen daraus.

11. Absorbierender Artikel, der ein nicht gewobenes Material nach einem der Ansprüche 6 bis 10 und einen absorbierenden Kern (4) umfaßt.

12. Absorbierender Artikel nach Anspruch 11, wobei das nicht gewobene Material die Abdeckung (3) des absorbierenden Artikels ist.

13. Absorbierender Artikel nach Anspruch 11, wobei das nicht gewobene Material die Rückschicht (5) des absorbierenden Artikels ist.

## Revendications

1. Procédé pour préparer une matière non tissée comportant une première surface (11) et une seconde surface (12) en face de la première surface (11), qui comprend séquentiellement :
a) l'application d'une composition adhésive (8) sur la première surface (11) de la matière non tissée selon un dessin;
b) la cuisson de la composition adhésive (8); et
c) la formation d'ouvertures dans la matière non tissée contenant la composition adhésive;
**caractérisé en ce que** ladite étape de formation d'ouvertures comprend un étape de formation d'une pluralité d'ouvertures (9) prenant naissance dans ladite seconde surface (12) de la matière non tissée, dans lequel les parois latérales desdites ouvertures (9) sont formées pour déborder hors de la seconde surface (12), faisant saillie vers l'extérieur à partir de la première surface.

2. Procédé selon la revendication 1, dans lequel ladite formation d'ouvertures est réalisée de manière à ce que lesdites ouvertures (9) soient formées dans les zones de la matière non tissée dans lesquelles la composition adhésive (8) n'a pas été appliquée.

3. Procédé selon la revendication 1 ou 2, dans lequel ladite formation d'ouvertures est réalisée de manière à ce qu'aucune desdites ouvertures, mais des surfaces d'appui gaufrées (15), soient formées dans les zones de la matière non tissée dans lesquelles la composition adhésive (8) a été appliquée, les surfaces d'appui gaufrées étant surélevées par rapport aux zones dans lesquelles la composition adhésive (8) n'a pas été appliquée.

4. Procédé selon la revendication 1, dans lequel la matière non tissée est un voile fibreux non tissé ou un film plastique (10).

5. Procédé selon la revendication 1, dans lequel la composition adhésive (8) comprend un additif sélectionné dans le groupe comprenant des matières hydrophiles, des matières hydrophobes, des polymères superabsorbants, des pigments et des combinaisons de ceux-ci.

6. Matière non tissée comprenant :
1) une première surface (11) et une seconde surface (12) en face de la première surface (11),
2) une composition adhésive (8) placée selon un dessin sur la première surface (11) de la matière non tissée, et
3) une pluralité d'ouvertures (9) dans ladite matière non tissée,
**caractérisée en ce que** ladite pluralité d'ouvertures (9) prend naissance dans ladite seconde surface (12) de la matière non tissée de sorte que les parois latérales desdites ouvertures (9) débordent hors de la seconde surface (12), faisant saillie vers l'extérieur à partir de la première surface.

7. Matière non tissée selon la revendication 7, dans laquelle la matière non tissée comprend la pluralité d'ouvertures (9) dans des zones dans lesquelles la composition adhésive (8) n'a pas été appliquée.

8. Matière non tissée selon la revendication 8, dans laquelle la matière non tissée ne comprend aucun trou, mais des surfaces d'appui gaufrées (15) dans des zones dans lesquelles la composition adhésive (8) a été appliquée, les surfaces d'appui gaufrées (15) étant surélevées par rapport aux zones de matière non tissée dans lesquelles la composition adhésive (8) n'a pas été appliquée.

9. Matière non tissée selon l'une quelconque des revendications 7 à 9, dans laquelle la matière non tissée est un voile fibreux non tissé ou un film plastique (10).

10. Matière non tissée selon l'une quelconque des revendications 7 à 9, dans laquelle la composition adhésive (8) comprend un additif sélectionné dans le groupe comprenant des matières hydrophiles, des matières hydrophobes, des polymères superabsorbants, des pigments et des combinaisons de ceux-ci.

11. Article absorbant comprenant une matière non tissée selon l'une quelconque des revendications 7 à 11 et un noyau absorbant (4).

12. Article absorbant selon la revendication 12, dans lequel ladite matière non tissée est la couverture (3) de l'article absorbant.

13. Article absorbant selon la revendication 12, dans lequel ladite matière non tissée est la feuille envers (5) de l'article absorbant.
